Europäisches Patentamt

European Patent Office

Office européen des brevets

(11) Publication number: **0 283 090 B1**

## EUROPEAN PATENT SPECIFICATION

(45) Date of publication of patent specification: **13.01.93**

(51) Int. Cl.⁵: **C08F 220/28**, C08F 220/04, A61L 15/00, //(C08F220/30, 220:04),(C08F220/04,220:30)

(21) Application number: **88200461.7**

(22) Date of filing: **10.03.88**

(54) **Water-swellable crosslinked polymers and process for the preparation thereof.**

(30) Priority: **11.03.87 GB 8705698**

(43) Date of publication of application:
**21.09.88 Bulletin 88/38**

(45) Publication of the grant of the patent:
**13.01.93 Bulletin 93/02**

(84) Designated Contracting States:
**BE DE FR GB NL**

(56) References cited:
**FR-A- 2 542 749**

**MONATSHEFTE FUR CHEMIE, vol. 112, 1981, Wien, New York GRUBER H. "Hydrophile Polymer-gele mit reaktiven Gruppen, 1. Mitt.: Herstellung und Polymerisation von Glucose- und Saccarosemethacrylaten" pages 273-285**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Graafland, Teunis**
**Badhuisweg 3**
**NL-1031 CM Amsterdam(NL)**

**Description**

The invention relates to water-swellable crosslinked vinyl saccharide terpolymers, to their preparation, and to shaped articles containing them.

Water-swellable, crosslinked polymeric materials which have a very high water-binding capacity are generally known as superabsorbents, and are increasingly being used in personal care products as well as in industrial and agricultural applications. Such superabsorbent polymeric materials are generally based on modified and/or crosslinked cellulose products as well as on certain crosslinked polyacrylates.

It has now been found that very useful water-swellable crosslinked vinyl saccharide terpolymers can be prepared, which match, and in certain aspects surpass the performance of known products, by employing in the preparation of these polymeric products water-soluble vinyl saccharide monomers in combination with water-soluble monomers carrying an acidic group and a relatively small amount of a crosslinking monomer, and, optionally, neutralizing the acidic groups.

Accordingly the invention provides water-swellable crosslinked vinyl saccharide terpolymers of:

a) 5-95 mol % of a water-soluble monovinyl saccharide monomer, being a mono- or disaccharide compound containing one polymerizable vinyl group per molecule;

b) 5-95 mol % of a water-soluble $\alpha,\beta$-olefinically unsaturated monomer, bearing an optionally neutralized carboxyl or sulphonic (-$SO_2OH$) group; and

c) 0.05-10 mol % of a divinyl unsaturated monomer,

the sum of the percentages sub a) and b) being 100 and the percentage sub c) being calculated on the sum of the percentages sub a) and b).

Products having less attractive performance properties are known from French Patent Application, published under No. 2 542 749, however, said products are based on a salified acrylic allyloligosaccharide copolymer, which consists in a chemically bound form of at least one allyloligosaccharide with a mean degree of substitution of less than 4 per molecule (i.e. the number of allyl groups per saccharide molecule) and at least one acrylic derivative with partly or totally salified carboxyl functions.

The term "monovinyl saccharide monomer" whenever used in the specification refers to mono- or disaccharide compounds containing one polymerizable vinyl group per molecule.

The preferred water-soluble monovinyl saccharide monomers contain a vinylcarbonyloxy group, such as a monomethacryloyl or a monoacryloyl group, and more preferably a monomethacryloyl group as they result in polymers which generally are hydrolytically more stable than the corresponding acrylic acid based polymers. Particularly preferred is 3-0-methacryloyl-D-glucose.

Examples of other polymerizable water-soluble monovinyl saccharide monomers bearing a vinylcarbonyloxy group are 3-0-acryloyl-D-glucose,6-0-acryloyl-D-galactose, 6-0-methacryloyl-D-galactose,1-0-acryloyl-L-sorbose, 1-0-methacryloyl-L-sorbose,1-0-acryloyl-mannose and 1-0-methacryloyl-mannose.

A method for the preparation of the polymerizable water-soluble vinylcarbonyloxy group containing monosaccharides has been disclosed by Black et al, Makromol. Chem. 117 (1968), 210.

Other suitable water-soluble monovinyl saccharide monomers contain a vinyloxy group, for example 3-0-vinyl-D-glucose, 6-0-vinyl-D-galactose and 1-0-vinyl-L-sorbose.

Methods for the preparation of the polymerizable water-soluble vinyloxy group-containing monosaccharides have been disclosed by e.g. Reppe et al, in Ann, 81 (1956) 601 and Watanabe and Colon in J. Am. Chem. Soc. 79 (1957) 2828.

The water-soluble $\alpha,\beta$-olefinically unsaturated monomer bearing an optionally neutralized carboxyl or a sulphonic (-$SO_2HO$) group suitably include acrylic acid, methacrylic acid, maleic acid, itaconic acid and 3-0-methacryloyl-D-gluconic acid. Preferably the $\alpha,\beta$-olefinically unsaturated monomer is a mono- or dicarboxylic acid. Methacrylic acid is a preferred $\alpha,\beta$-olefinically unsaturated monocarboxylic acid.

Suitable divinyl unsaturated monomers include compounds such as a divinylbenzene, a divinyltoluene, a divinylxylene, a divinylpyridine, divinyl ketone and divinylsulfone; divinyl ethers such as the divinyl ether of ethylene glycol, divinyl ethers of polyethylene glycols; diesters derived from two mol of a monoethylenically unsaturated carboxylic acid and a diol, such as ethylene glycol diacrylate, ethylene glycol dimethacrylate, polyethylene glycol diacrylate, and polyethylene glycol dimethacrylate; and monomers such as N,N'-methylenebisacrylamide. N,N'-methylenebisacrylamide is a preferred divinyl unsaturated monomer.

The invention further provides a process for the preparation of a crosslinked vinyl saccharide terpolymer described hereinbefore, wherein:

a) 5-95 mol % of a water-soluble monovinyl saccharide monomer, being a mono- or disaccharide compound containing one polymerizable vinyl group per molecule;

b) 5-95 mol % of water-soluble $\alpha,\beta$-olefinically unsaturated monomer, bearing an optionally neutralized carboxyl or sulphonic (-$SO_2OH$) group; and

c) 0.05-10 mol % of a divinyl unsaturated monomer,

the sum of the percentages sub a) and b) being 100 and the percentage sub c) being calculated on the sum of the percentages sub a) and b), is polymerized in an aqueous medium in the presence of a free-radical initiator at a temperature in the range of from 0 to 95 °C.

Although the crosslinked polymers described hereinbefore demonstrate a modest degree of waterabsorbancy when in the acidic form, the water absorption increases very strongly upon neutralization of said crosslinked polymers. The invention therefore also provides neutralized crosslinked vinyl saccharide terpolymers where the acidic groups in the terpolymers according to the present invention have been neutralized.

The expression "neutralized terpolymer" herein refers to a product wherein at least 50 mol % of the acidic groups have been neutralized employing a neutralizing agent as described hereinafter. Neutralizing agents which may be employed to arrive at the neutralized terpolymers include alkali metal hydroxides, ammonia, tertiary amines and alkanolamines. Alkali metal hydroxides are preferred neutralizing agents, particularly sodium hydroxide.

Therefore, the invention also provides a process for the preparation of a neutralized crosslinked vinyl saccharide terpolymer as described hereinbefore wherein

a) 5-95 mol % of a water-soluble monovinyl saccharide monomer being a mono- or disaccharide compound containing one polymerizable vinyl group per molecule;

b) 5-95 mol % of a water-soluble $\alpha,\beta$-olefinically unsaturated monomer, bearing an optionally neutralized carboxyl or sulphonic ($-SO_2OH$) group; and

c) 0.05-10 mol % of a divinyl unsaturated monomer,

the sum of the percentages sub a) and b) being 100 and the percentage sub c) being calculated on the sum of the percentages sub a) and b),

is polymerized in an aqueous medium in the presence of a free-radical initiator at a temperature in the range of from 0 to 95 °C, and wherein the acidic groups are neutralized before or after the polymerization.

The free-radicals to be used in the process of the present invention may originate from compounds which are capable of generating free-radicals via a reduction-oxidation reaction, so called redox initiators, as well as from compounds which are capable of generating free-radicals by decomposition, e.g. upon heating thereof. Redox initiator systems have been described e.g. in Prog. Polym. Sci. Vol. 8, pp. 61-131 (1982). Preferred redox initiator systems are selected from the group comprising a peroxide, a persulphate, a peroxydiphosphate or a permanganate type of oxidant, and a reducing agent.

Preferred reducing agents are based on a reducing acid of sulphur.

Compounds which are capable of generating free-radicals via a decomposition reaction, have been described e.g. in J. Macr. Sci. Rev. Macr. Chem., C 20(1), 149-205 (1981) and include azo and diazo compounds, organic peroxides, hydroperoxide, peroxydicarbonate and persulphate type of free-radical initiators, and hydrogen peroxide.

In the process for the preparation of the crosslinked polymers of the present invention 2,2'-azobisisobutyronitrile is a preferred free-radical initiator.

Although the amount of free-radical or redox initiator to be used may vary over wide ranges there is a preference to employ said initiator in a molar ratio of free radical initiator or oxidant compound of the redox initiator to total monomer of not higher than 1:100 and more preferably not higher than 1:500.

When employing a free-radical initiator of low water-solubility it is advantageous to add said initiator as a solution in a water-miscible solvent such as acetone.

Although the process for preparing the crosslinked polymers of the present invention encompasses the neutralization of the acidic group before polymerization, i.e. employing monomers wherein the acidic groups have been neutralized, as well as neutralization upon completion of the polymerization step, there is a preference for the latter procedure.

It will be understood by those skilled in the art that the neutralization of the acidic groups in the crosslinked polymer may be relatively slow, in view of the polymer being insoluble. It may therefore be beneficial, to achieve the highest degree of neutralization, to employ a molar excess of neutralizing agent over the acidic groups as well as conducting said neutralization in the presence of additional water to lower the overall viscosity of the aqueous medium in which the crosslinked polymer is present. The neutralizing agents which may be employed in the process for the preparation of the neutralized crosslinked polymers are the same as those mentioned hereinbefore.

The neutralized crosslinked polymers may be isolated by precipitation in an excess of a suitable organic liquid e.g. an alcohol such as ethanol and 2-propanol. The precipitate may subsequently be washed employing an alcohol as mentioned hereinbefore and finally dried.

Mixtures of water-soluble monovinyl saccharide monomers and/or mixtures of water-soluble monomers

3

carrying an acidic group and/or mixtures of divinyl unsaturated monomers may also be used.

The invention will be further illustrated by the following Examples from which the following information is provided:

a Abbreviations used:

| Code | Chemical name |
|------|---------------|
| MG | 3-0-methacryloyl-D-glucose |
| MA | methacrylic acid |
| MBA | N,N'-methylenebisacrylamide |
| AIBN | 2,2'-azobisisobutyronitrile |
| IPA | 2-propanol |

b In all the experimental work double-distilled demineralized water was used.

c Preparation of MG

4 g of 1,2:5,6-di-0-isopropylidene-3-0-methacryloyl-D-glucose (DIMG), 25 mg of p-methoxyphenol and 30 ml 0.5 N HCl were introduced into a 100 ml glass reactor equipped with a glass stirrer, a thermometer and a reflux condensor, and subsequently the reactor was placed in a thermostated oil-bath. The reactor contents were heated to 70°C with continuous stirring, and maintained at this temperature until the contents had become homogeneous (1-2 h). After cooling to room temperature the reactor contents were transferred to a separatory funnel. To remove the p-methoxyphenol, the 3-0-methacryloyl-D-glucose solution was treated with 50 ml diethyl ether and after phase separation had occurred the organic phase was removed. This extraction procedure was repeated four times. Subsequently the aqueous solution was transferred to the above-mentioned 100 ml glass reactor and a pressure slightly below atmospheric was applied to remove the last traces of diethyl ether. Finally the solution was neutralized to a pH of 7 with 1.0 N NaOH, which procedure was carefully monitored by pH measurement. A solids determination indicated that the MG content of the aqueous solution was 8 %w (=0.32 mol/l).

d Preparation of MA solution

100 g MA containing 200 ppm hydroquinone was distilled over copper wire at a pressure of 0.09 bar, cooled with ice and stored under nitrogen. The aqueous MA solution was prepared immediately before use by dissolving 8 g MA in 92 g of water.

e Preparation of MBA solution

2 g MBA, a white powder, was dissolved in 98 g of water immediately before use.

f Synthetic urine

In a number of methods of assessing the absorption capacity of the crosslinked polymers a synthetic urine was used, having the following composition:

| | |
|------|------|
| Water | 2894 g |
| Sodium chloride | 30 g |
| Ureum | 72 g |
| Magnesium sulphate hydrate($H_2O$) | 1.8 g |
| Calcium acetate hydrate($0.5H_2O$) | 2.1 g |

In the tests employed to assess the absorption capacity of the polymers, a commercially available crosslinked polyacrylate Feinstaub 96 (Trade name) ex Stockhausen was included as a reference.

Example I

Preparation of neutralized crosslinked polymers.

Crosslinked vinyl saccharide polymers were prepared via a free-radical initiated polymerization in the aqueous phase employing MG, MA and MBA in the amounts as indicated in Table 1 hereinafter. MG and MA were introduced into a 100 ml glasreactor as an 8 % w solution in water, while MBA was added as a 2 % w solution in water. The reaction contents were stripped with oxygen-free nitrogen to remove any oxygen present and subsequently the required amount of AIBN (2 mg AIBN/ ml acetone) was added to arrive at an AIBN/total monomer molar ratio 1:2000. After a further stripping cycle the reactor was placed in a thermostated oil bath of 40°C for 48 hours. After polymerization the jelly type reactor contents were transferred to a 500 ml bottle and 1.0 M aqueous NaOH was added in an amount which was more than sufficient to neutralize all the acidic groups. To achieve the highest possible degree of neutralization, the bottles were placed on a roller table for at least 24 hours. The degree of neutralization was checked with a paper pH indicator. The reactor contents were subsequently poured out into an excess (approximately 200 ml) of IPA and after thorough stirring, phase separation was allowed to set in. After isolating the precipitate it was again treated with IPA and after renewed isolation the precipitate was dried at 50°C under sub-atmospheric pressure in a nitrogen atmosphere, wich resulted in a white amorphous solid.

Example II.

Determining the absorption capacity of neutralized crosslinked vinyl saccharide polymers via a centrifuge method.

The absorption capacity of the crosslinked polymers as prepared in experiments 1-16 of Example I was determined by contacting 10 to 500 mg of polymer with an excess of liquid of the type as indicated in Table 2 hereinafter (approx 37 ml), in a calibrated 50 ml centrifuge tube during 10 minutes. Subsequently the tube was placed in a centrifuge for 20 minutes at 2700 revolutions per minute (RPM). With most of the samples this resulted in a two phase system, wherein the volume of the lower phase which contained the crosslinked polymer, was a measure for the absorption capacity of the polymer. The values, are given in Table 2.

Example III.

Determining the water absorption capacity of neutralized crosslinked vinyl saccharide polymers via filtration.

100 mg Samples of the polymers as prepared in experiments 1-16 of Example I were contacted with water (100 or 300 ml) during 10 minutes while the polymer from experiment 10 was also contacted with water for 40 hours. Subsequently the aqueous medium containing the crosslinked polymer was transferred to a glass filter A (porosity 1, volume 125 ml, diameter 60 mm) or B (porosity 1, volume 500 ml, diameter 120 mm) which had been placed on a Buchner flask, and filtrated during 10 minutes at a pressure of 0.92 bar. The weight of the gel on the filter is a measure for the water absorption capacity of the crosslinked polymers. The results are given in Table 3.

Example IV.

Determining the absorption capacity of neutralized crosslinked vinyl saccharide polymers via the "tea bag" method.

The water absorption capacity of the crosslinked polymers as prepared in experiments 1-16, as well as the urine absorption capacity of those from experiments 9-12 were determined via the so called teabag method described hereinafter. Powdered crosslinked polymer (100 respectively 500 mg) was introduced into a 10 x 20 cm teabag prepared from 250 mesh nylon gauze. The powdered polymer was spread out evenly over the internal surface of the teabag to ensure the best possible absorption conditions. The polymer containing teabag was soaked in an excess (approximately 4 to 1) of water or synthetic urine for a period of time as indicated in Table 4 hereinafter. After removal from the beaker containing the test liquid the bags were suspended in air for 10 minutes to allow the excess water to leak out, which was followed by weighing the swollen teabag. The weight increase of the wet tea bags was taken as a measure for the absorption capacity of the crosslinked polymers, and is given in Table 4.

5

Table 1.

| Experiment No. | MG mmol | MA mmol | MBA mmol | AIBN sol ml | 1.0 N NaOH ml |
|---|---|---|---|---|---|
| 1 | 19.3 | 4.83 | 0.16 | 1.0 | 5 |
| 2 | 19.3 | 4.83 | 0.32 | 1.0 | 5 |
| 3 | 19.3 | 4.83 | 0.64 | 1.0 | 5 |
| 4 | 19.3 | 4.83 | 1.60 | 1.0 | 5 |
| 5 | 19.3 | 12.8 | 0.18 | 1.3 | 13 |
| 6 | 19.3 | 12.8 | 0.36 | 1.3 | 13 |
| 7 | 19.3 | 12.8 | 0.72 | 1.3 | 13 |
| 8 | 19.3 | 12.8 | 1.80 | 1.3 | 13 |
| 9 | 22.6 | 22.6 | 0.12 | 1.85 | 23 |
| 10 | 22.6 | 22.6 | 0.23 | 1.85 | 23 |
| 11 | 22.6 | 22.6 | 0.35 | 1.85 | 23 |
| 12 | 22.6 | 22.6 | 0.46 | 1.85 | 23 |
| 13 | 19.3 | 29 | 0.22 | 2.0 | 30 |
| 14 | 19.3 | 29 | 0.45 | 2.0 | 30 |
| 15 | 19.3 | 29 | 0.89 | 2.0 | 30 |
| 16 | 19.3 | 29 | 2.23 | 2.0 | 30 |

Table 2

| Polymer from experiment No. | Absorption capacity, g per g polymer, for | |
| --- | --- | --- |
| | water. | synthetic urine |
| 1 | 680±70 | – |
| 2 | 870±70 | – |
| 3 | 580±20 | – |
| 4 | 228± 4 | – |
| 5 | * | – |
| 6 | 1060±20 | – |
| 7 | 860±20 | – |
| 8 | 240±11 | – |
| 9 | – | 52 |
| 10 | – | 44 |
| 11 | – | 48 |
| 12 | – | 44 |
| 13 | * | – |
| 14 | 890±10 | – |
| 15 | 760± 8 | – |
| 16 | 215±10 | – |
| Reference sample. | – | 46 |

* No definite phase separation observed.

Table 3.

| Polymer from experiment No. | Absorption time. | Filter type. | Absorption capacity, g water per g polymer |
|---|---|---|---|
| 1 | 10 min | A | 555 |
| 2 | " | " | 568 |
| 3 | " | " | 290 |
| 4 | " | " | 146 |
| 5 | " | " | 895 |
| 6 | " | " | 840 |
| 7 | " | " | 356 |
| 8 | " | " | 139 |
| 9 | " | B | 695 |
| 10 | " | " | 1231 |
| | 40 h | " | 2220 |
| 11 | 10 min | " | 1068 |
| 12 | " | " | 834 |
| 13 | " | A | 623 |
| 14 | " | " | 672 |
| 15 | " | " | 332 |
| 16 | " | " | 120 |
| Reference sample } | " | B | 246 |
| | 40 h | " | 253 |

8

Table 4.

| Polymer from experiment | Sample size mg | Absorption time | Absorption capacity, g per g polymer, | |
|---|---|---|---|---|
| | | | water | synth. urine |
| 1 | 500 | 10 min | 254 | - |
| 2 | " | " | 312 | - |
| 3 | " | " | 216 | - |
| 4 | " | " | 152 | - |
| 5 | " | " | 384 | - |
| 6 | " | " | 390 | - |
| 7 | " | " | 277 | - |
| 8 | " | " | 167 | - |
| 9 | 100/500* | 30 min | 615 | 57 |
| | " | 15 h | 965 | 79 |
| 10 | " " | 30 min | 950 | 48 |
| | " | 15 h | 1230 | 57 |
| 11 | " " | 30 min | | 35 |
| | " | 15 h | 1110 | 52 |
| 12 | " " | 30 min | 820 | 42 |
| | " | 15 h | 1005 | 50 |
| 13 | 500 | 10 min | 420 | - |
| 14 | " | " | 237 | - |
| 15 | " | " | 281 | - |
| 16 | " | " | 142 | - |
| Reference sample } | 100/500* " / " | 30 min 15 h | 266 294 | 35 31 |

* Sample size for urine absorption test.

From the results as given in Tables 1-4 it can be concluded that the neutralized crosslinked terpolymers of the present invention demonstrate very high absorption capacity values for water, which capacity appears to be related to the monomer composition employed for the preparation of the crosslinked polymers, i.e. to the MG/MA molar ratio as well as to the amount of MBA employed. Although the absorption capacity values obtained via the individual test methods differ greatly, they all support the fact that neutralized crosslinked polymers derived from a monomer composition comprising:

40-60 mol % water-soluble monovinyl saccharide monomer

40-60 mol % water-soluble monomer bearing a carboxyl or sulphonic group, and

0.25-2.5 mol % of a divinyl unsaturated monomer are found to have the highest water absorption values and are therefore preferred neutralized crosslinked vinyl saccharide polymers.

The performance of the polymers of the present invention makes them potentially very suitable for outlets where a high water absorption is an important requisite, i.e. in personal care products as well as in industrial and agricultural applications.

**Claims**

1. Water-swellable crosslinked vinyl saccharide terpolymers of:

a) 5-95 mol % of a water-soluble monovinyl saccharide monomer, being a mono- or disaccharide compound containing one polymerizable vinyl group per molecule,

b) 5-95 mol % of a water-soluble $\alpha,\beta$-olefinically unsaturated monomer bearing an optionally neutralized carboxyl or sulphonic ($-SO_2OH$) group, and

9

c) 0.05-10 mol % of a divinyl unsaturated monomer,
the sum of the percentages sub a) and b) being 100 and the percentage sub c) being calculated on the sum of the percentages sub a) and b).

2. Terpolymers as claimed in claim 1, wherein the monovinyl saccharide monomer contains a vinylcarbonyloxy group.

3. Terpolymers as claimed in claim 2, wherein the vinylcarbonyloxy group is a monomethacryloyl group.

4. Terpolymers as claimed in claim 3, wherein the monovinyl saccharide is 3-0-methacryloyl-D-glucose.

5. Terpolymers as claimed in any one of the preceding claims, wherein the monomer carrying a carboxyl group is an $\alpha,\beta$-olefinically unsaturated mono- or dicarboxylic acid.

6. Terpolymers as claimed in claim 5, wherein the $\alpha,\beta$-olefinically unsaturated monocarboxylic acid is methacrylic acid.

7. Terpolymers as claimed in any one of the preceding claims, wherein the divinyl unsaturated monomer is N,N'-methylenebisacrylamide.

8. Terpolymers as claimed in any one of the preceding claims which are terpolymers of:
a) 40-60 mol % of the water-soluble monovinyl saccharide monomer,
b) 40-60 mol % of the water-soluble monomer carrying a neutralized carboxyl or sulphonic group, and
c) 0.25-2.5 mol % of the divinyl unsaturated monomer,
the sum of the mol percentages sub a) and b) being 100 and the percentage sub c) being calculated on the sum of the mol percentage sub a) and b).

9. Terpolymers as claimed in claim 8, wherein the carboxyl or sulphonic group has been neutralized with an alkali metal hydroxide.

10. A process for the preparation of a crosslinked vinyl saccharide terpolymer as claimed in any one of claims 1-7 wherein
a) 5-95 mol % of a water-soluble monovinyl saccharide monomer, being a mono- or disaccharide compound containing one polymerizable vinyl group per molecule,
b) 5-95 mol % of a water-soluble $\alpha,\beta$-olefinically unsaturated monomer, bearing an optionally neutralized carboxyl or sulphonic (-SO$_2$OH) group, and
c) 0.05-10 mol % of a divinyl unsaturated monomer,
the sum of the mol percentages sub a) and b) being 100 and the percentage sub c) being calculated on the sum of the mol percentage sub a) and b), is polymerized in an aqueous medium in the presence of a free-radical initiator at a temperature in the range of from 0 to 95 °C.

11. A process for the preparation of a neutralized crosslinked vinyl saccharide terpolymer as claimed in any one of claims 1-9, wherein
a) 5-95 mol % of a water-soluble monovinyl saccharide monomer, being a mono- or disaccharide compound containing one polymerizable vinyl group per molecule,
b) 5-95 mol % of a water-soluble $\alpha,\beta$-olefinically unsaturated monomer, bearing an optionally neutralized carboxyl or sulphonic (-SO$_2$OH) group, and
c) 0.05-10 mol % of a divinyl unsaturated monomer,
the sum of the percentages sub a) and b) being 100 and the percentage sub c) being calculated on the sum of the percentage sub a) and b), is polymerized in an aqueous medium in the presence of a free-radical initiator at a temperature in the range of from 0 to 95 °C and wherein the acidic groups are neutralized before or after the polymerization.

12. A process as claimed in claim 10 or 11 wherein the free-radical initiator is 2,2'-azobisisobutyronitrile.

13. Shaped articles which consist at least partly of terpolymers as claimed in any one of claims 1 to 9.

**Patentansprüche**

1. In Wasser quellfähig vernetzte Vinylsaccharid-Terpolymere aus:
   a) 5 bis 95 Mol% eines wasserlöslichen Monovinylsaccharid-Monomers in Form einer Mono- oder Disaccharidverbindung, die eine polymerisierbare Vinylgruppe im Molekül enthält,
   b) 5 bis 95 Mol% eines wasserlöslichen $\alpha,\beta$-olefinisch ungesättigten Monomers mit einer gegebenenfalls neutralisierten Carboxyl-oder Sulfonsäuregruppe ($-SO_2OH$) und
   c) 0,05 bis 10 Mol% eines divinylartig ungesättigten Monomeren,
   wobei die Summe der Prozentanteile von a) und b) 100 beträgt und der Prozentanteil von c) sich auf die Summe der Prozentanteile von a) und b) bezieht.

2. Terpolymere, wie in Anspruch 1 beansprucht, wobei das Monovinylsaccharid-Monomere eine Vinylcarbonyloxygruppe enthält.

3. Terpolymere, wie in Anspruch 2 beansprucht, wobei die Vinylcarbonyloxygruppe eine Monomethacryloylgruppe ist.

4. Terpolymere, wie in Anspruch 3 beansprucht, wobei das Monovinylsaccharid die Verbindung 3-O-Methacryloyl-D-glucose ist.

5. Terpolymere nach irgendeinem der vorhergehenden Ansprüche, wobei das eine Carboxylgruppe aufweisende Monomer eine $\alpha,\beta$-olefinisch ungesättigte Mono- oder Dicarbonsäure ist.

6. Terpolymere, wie in Anspruch 5 beansprucht, wobei die $\alpha,\beta$-olefinisch ungesättigte Monocarbonsäure die Methacrylsäure ist.

7. Termpolymere, wie in irgendeinem der vorhergehenden Ansprüche beansprucht, wobei das divinylartig ungesättigte Monomer die Verbindung N,N'-Methylenbisacrylamid ist.

8. Terpolymere, wie in irgendeinem der vorhergehenden Ansprüche beansprucht, wobei die Terpolymere aus:
   a) 40 bis 60 Mol% des wasserlöslichen Monovinylsaccharid-Monomers,
   b) 40 bis 60 Mol% des eine Carboxyl- oder Sulfonsäuregruppe aufweisenden wasserlöslichen Monomers und
   c) 0,25 bis 2,5 Mol% des divinylartig ungesättigten Monomers bestehen
   und sich die Summe der Prozentanteile von a) und b) zu 100 ergänzt und der Prozentanteil von c) sich auf die Summe der Prozentanteile von a) und b) bezieht.

9. Terpolymere, wie in Anspruch 8 beansprucht, wobei die Carboxyl- oder Sulfonsäuregruppe in mittels eines Alkalihydroxids neutralisierter Form vorliegt.

10. Ein Verfahren zur Herstellung eines vernetzten Vinylsaccharid-Terpolymers, wie in irgendeinem der Ansprüche 1 bis 7 beansprucht, in dem
    a) 5 bis 95 Mol% eines wasserlöslichen Monovinylsaccharid-Monomers in Form einer Mono- oder Disaccharidverbindung, die eine polymerisierbare Vinylgruppe im Molekül enthält,
    b) 5 bis 95 Mol% eines wasserlöslichen $\alpha,\beta$-olefinisch ungesättigten Monomers mit einer gegebenenfalls neutralisierten Carboxyl- oder Sulfonsäuregruppe ($-SO_2OH$) und
    c) 0,05 bis 10 Mol% eines divinylartig ungesättigten Monomers,
    wobei die Summe der Prozentanteile von a) und b) 100 beträgt und der Prozentanteil von c) sich auf die Summe der Prozentanteile von a) und b) bezieht,
    in einem wäßrigen Medium in Anwesenheit eines freie Radikale bildenden Initiators bei einer Temperatur im Bereich von 0 bis 95 ° C miteinander polymerisiert werden.

11. Ein Verfahren zur Herstellung eines neutralisierten vernetzten Vinylsaccharid-Terpolymers, wie in irgendeinem der Ansprüche 1 bis 9 beansprucht, in dem
    a) 5 bis 95 Mol% eines wasserlöslichen Monovinylsaccharid-Monomers in Form einer Mono- oder Disaccharidverbindung, die eine polymerisierbare Vinylgruppe im Molekül enthält,
    b) 5 bis 95 Mol% eines wasserlöslichen $\alpha,\beta$-olefinisch ungesättigten Monomers mit einer gegebenenfalls neutralisierten Carboxyl- oder Sulfonsäuregruppe (-SO$_2$OH) und

   c) 0,05 bis 10 Mol% eines divinylartig ungesättigten Monomeren,

wobei die Summe der Prozentanteile von a) und b) 100 beträgt und der Prozentanteil von c) sich auf die Summe der Prozentanteile von a) und b) bezieht,

in einem wäßrigen Medium in Anwesenheit eines freie Radikale bildenden Initiators bei einer Temperatur im Bereich von 0 bis 95 °C miteinander polymerisiert werden und die sauren Gruppen vor oder nach der Polymerisation neutralisiert werden.

**12.** Ein Verfahren, wie in Anspruch 10 oder 11 beansprucht, wobei der freie Radikale bildende Initiator 2,2'-Azobisisobutyronitril ist.

**13.** Geformte Gegenstände, die mindestens teilweise aus Terpolymeren, wie in irgendeinem der Ansprüche 1 bis 9 beansprucht, bestehen.

**Revendications**

**1.** Polymères ternaires de saccharides vinyliques réticulés et gonflables dans l'eau, comprenant :

   (a) 5 à 95 moles% d'un monomère de saccharide monovinylique soluble dans l'eau, qui est un composé mono- ou di-saccharide contenant un groupe vinyle polymérisable par molécule,

   (b) 5 à 95 moles% d'un monomère hydrosoluble à insaturation alpha,bêta-oléfinique portant un groupe carboxyle ou sulfonique facultativement neutralisé (-SO$_2$OH) et

   (c) 0,05 à 10 moles% d'un monomère divinylique insaturé,

   la somme des pourcentages de (a) et (b) étant de 100 et le pourcentage de (c) étant calculé par rapport à la somme des pourcentages (a) et (b).

**2.** Polymères ternaires selon la revendication 1, dans lesquels le monomère saccharide monovinylique contient un groupe vinylcarbonyloxy.

**3.** Polymères ternaires selon la revendication 2, dans lesquels le groupe vinylcarbonyloxy est un groupe monométhacryloyle.

**4.** Polymères ternaires selon la revendication 3, dans lesquels le saccharide monovinylique est le 3-0-méthacryloyl-D-glucose.

**5.** Polymères ternaires selon l'une quelconque des revendications précédentes dans lesquels le monomère portant un groupe carboxyle est l'acide mono- ou dicarboxylique à insaturation alpha,bêta-oléfinique.

**6.** Polymères ternaires selon la revendication 5, dans lesquels l'acide monocarboxylique à insaturation alpha,bêta-oléfinique est l'acide méthacrylique.

**7.** Polymères ternaires selon l'une quelconque des revendications précédentes, dans lesquelles le monomère divinylique insaturé est le N,N'-métttylènebisacrylamide.

**8.** Polymères ternaires selon l'une quelconque des revendications précédentes, qui sont les polymères ternaires de :

   a) 40 à 60 moles% d'un monomère hydrosoluble de saccharide monovinylique ;

   b) 40 à 60 moles% de monomère hydrosoluble portant un groupe carboxyle ou sulfonique neutralisé, et

   c) 0,25 à 2,5 moles% de monomère divinylique insaturé, la somme des pourcentages molaires de (a) et (b) étant de 100 et le pourcentage de (c) étant calculé sur la somme des pourcentages molaires de (a) et (b).

**9.** Polymères ternaires selon la revendication 8, dans lesquels le groupe carboxyle ou sulfonique a été neutralisé avec un hydroxyde de métal alcalin.

**10.** Procédé de préparation d'un polymère ternaire réticulé de saccharide vinylique selon l'une quelconque des revendications 1 à 7, selon lequel : on polymérise en milieu aqueux et en présence d'un activant radicalaire à une température de 0 à 95°C

(a) de 5 à 95 moles% d'un monomère hydrosoluble de saccharide monovinylique qui est un mono- ou di-saccharide contenant un groupe vinylique polymérisable par molécule,

(b) de 5 à 95 moles% d'un monomère hydrosoluble à insaturation alpha,bêta-oléfinique portant un groupe carboxyle ou sulfonique (-$SO_2OH$) facultativement neutralisé et

(c) de 0,05 à 10 moles% d'un monomère divinylique insaturé, la somme des pourcentages molaires de (a) et (b) étant 100 et le pourcentage de (c) étant calculé sur la somme des pourcentages molaires de (a) et (b).

11. Procédé de préparation d'un polymère ternaire réticulé et neutralisé de vinylsaccharide selon l'une quelconque des revendications 1 à 9, selon lequel : on polymérise en milieu aqueux et en présence d'un activant radicalaire à une température de 0 à 95 ° C

(a) de 5 à 95 moles% d'un monomère hydrosoluble de saccharide monovinylique qui est un mono- ou di-saccharide contenant un groupe vinylique polymérisable par molécule,

(b) de 5 à 95 moles% d'un monomère hydrosoluble à insaturation alpha,bêta-oléfinique portant un groupe carboxyle ou sulfonique (-$SO_2OH$) facultativement neutralisé et

(c) de 0,05 à 10 moles% d'un monomère divinylique insaturé, la somme des pourcentages molaires de (a) et (b) étant 100 et le pourcentage de (c) étant calculé sur la somme des pourcentages molaires de (a) et (b), et selon lequel les groupes acides sont neutralisés avant ou après la polymérisation.

12. Procédé selon la revendication 10 ou 11, dans lequel l'activant radicalaire est le 2,2'-azobisisobutyronitrile.

13. Articles façonnés qui consistent au moins partiellement en polymères ternaires selon l'une quelconque des revendications 1 à 9.